# EUROPEAN PATENT SPECIFICATION

(11) **EP 0 701 420 B1**
(45) Date of publication and mention of the grant of the patent: **12.11.1997**
(21) Application number: 95904292.0
(22) Date of filing: 08.12.1994
(51) Int. Cl.: A61F 2/34

(54) **ACETABULAR SHELL HAVING SCREW HOLES WITH BREAKOUT SEALS**
HÜFTGELENKPFANNE MIT FÜR DIE VERSCHRAUBUNGVORGESCHENEN,AUFBRECHBAREN SACKLÖCHERN
COQUE ACETABULAIRE A TROUS A VIS DOTES D'ELEMENTS D'OBTURATION APTES A ETRE ROMPUS

(30) Priority: 10.12.1993 US 165249
(43) Date of publication of application: 20.03.1996
(73) Proprietor: SULZER ORTHOPEDICS INC., Austin Texas 78752 (US)
(72) Inventor: MISER, John, D., Round Rock, TX 78664 (US)
(74) Representative: Bayliss, Geoffrey Cyril
(86) International application number: US9414123
(87) International publication number: WO9515734

(56) References cited:
- EP-A- 0 499 475
- US-A- 4 955 325

## Description

### Technical Field

The present invention relates generally to implantable prostheses for human joint replacement, and relates more particularly to an acetabular cup for a hip prosthesis.

### Background Information

A total hip prostheses includes a femoral component and an acetabular cup component. The femoral component has a stem for receipt and fixation within the femoral canal at a resected proximal end of the femur and has a spherical head that may be integral with or removably attached to the stem. The acetabular cup has an outer, generally spherical surface for receipt within a reamed acetabulum and has an inner bearing cavity for receiving the head of the femoral component. The head articulates relative to the bearing cavity such that the total hip prosthesis restores a normal range of motion to an otherwise diseased or damaged hip joint.

One known general configuration for an acetabular cup involves an outer acetabular shell made of a biocompatible metal such as titanium, and a bearing insert made of a biocompatible plastic such as high-density polyethylene. The acetabular shell has a generally hemispherical cup shape, defined by a generally spherical outer surface and a generally spherical inner surface with a shell wall between the inner and outer surfaces. The inner wall defines a generally hemispherical shell cavity having an opening for receipt of the bearing insert. The bearing insert has a generally spherical outer surface dimensioned to be received and fixed within the shell cavity of the acetabular shell, and has a bearing cavity facing outwardly for receipt of the head of the femoral component.

Acetabular shells of such known construction can be affixed to the bone of the patient's acetabulum by bone screws or bone cement. If bone screws are selected, the screws are inserted through screw holes in the acetabular shell prior to insertion of the bearing insert into the shell. The shell can also be affixed by a combination of bone screws and bone cement. The acetabular shell will often be provided with a plurality of screw holes in excess of the number of bone screws that would typically be used by the implanting physician, so as to provide a selection of screw sites. Thus, some of the provided screw holes may be used while others are left open, or, in the case where no bone screws are used, all of the screw holes are left open.

Although the bearing insert is usually designed to be received in non-articulating relationship within the acetabular shell, it is believed that a small amount of relative motion between the bearing insert and the acetabular shell nevertheless occurs under the varying load that the acetabular cup is subjected to during use. Such small relative motion, or micro-motion, may result in wear at the interface between the bearing insert and acetabular shell, resulting in the generation of polyethylene or metal debris. It has been suggested that such debris can migrate out of the acetabular cup and come in contact with bone, possibly resulting in osteolysis, which ultimately can lead to bone resorption and possible loosening of the acetabular prosthesis. One apparent pathway for the migration of debris out of the acetabular shell is through open screw holes.

It would be desirable to provide an acetabular shell, designed for use with a bearing insert, that is provided with screw holes for receipt of bone screws, wherein unused screw holes would be occluded to prevent migration through such unused screw holes of wear debris generated within the acetabular shell. This and other desirable features are provided by the present invention.

### Disclosure of the Invention

An acetabular shell is provide that can receive bone screws, if desired. Screw holes are provided that are inherently occluded by an integral seal, which seal can be broken out to render the screw hole as a through hole far receipt of a bone screw therethrough. If desired, the seal can be left in place if the screw hole is not to be used to receive a bone screw, thereby preventing migration of wear debris from the interior of the acetabular shell to the exterior of the shell through an open, unused screw hole.

In accordance with one aspect of the invention, there is provided a generally hemispherical cup-shaped shell having an inner surface and an outer surface defining a shell wall therebetween. The shell wall has at least one blind hole in the inner surface thereof. The blind hole terminates sufficiently close to the outer surface of the shell to leave a thin seal integral with the shell wall that occludes and separates the blind hole from the exterior of the shell. The seal has at least a portion thereof that is sufficiently thin that the seal can be broken out of the shell wall to render the blind hole as a through hole communicating from the interior to the exterior of the shell.

It is an object of the present invention to provide an acetabular shell that presents the physician with the choice of using bone screws or not to secure the shell in the acetabulum, while providing for occlusion of screw holes that are not selected to receive a bone screw so that migration of wear debris from the interior of the shell to the exterior through open, unused screw holes is prevented.

Other objects and advantages of the present invention will be apparent from the following descriptions of a preferred embodiment with reference to the drawings.

### Brief Description of the Drawings

FIG. 1 is a side view of an acetabular shell constructed in accordance with the present invention, shown partially in section.

FIG. 2 is an axial view of the acetabular shell of FIG. 1 looking into the cavity of the acetabular shell.

FIG. 3 is an enlarged cross-section of a portion of the shell wall of the acetabular shell of FIG. 1, showing a section through a screw hole.

### Best Mode for Carrying out the Invention

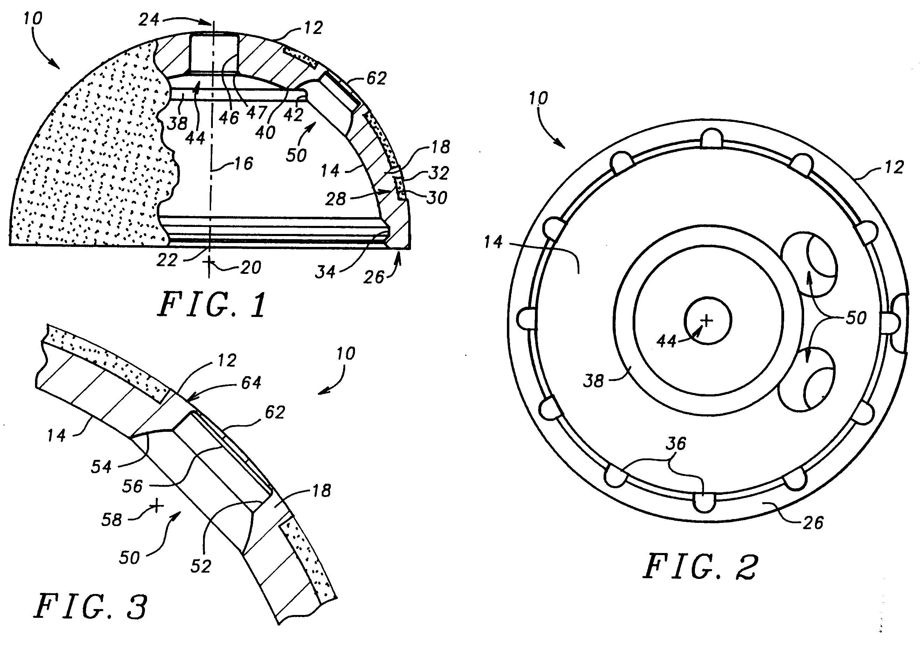

Referring in particular to FIGS. 1 and 2, there is illustrated an acetabular shell 10 generally shaped as a hemispherical cup defined by an outer hemispherical surface 12 and an inner hemispherical surface 14 having centers that lie on a common axis 16. Inner and outer surfaces 12 and 14 define therebetween a shell wall 18. Preferably, the center 20 of inner surface 14 is offset axially from the center 22 of outer surface 12 such that shell wall 18 is of non-constant thickness. In particular, shell wall 18 is thicker near the apex 24 than near the rim 26.

Shell wall 18, preferably constructed of solid titanium metal, has areas of reduced thickness 28 due to recesses 30 in the outer surface 12. Recesses 30 are filled with a porous titanium coating 32 to a level substantially flush with outer surface 12. The porous coating 32 provides a surface that promotes adhesion of bone cement to acetabular shell 10 and that is capable of promoting and accepting ingrowth of bone therein in non-cemented applications.

An annular groove 34 is located in inner surface 14 proximate rim 26 for the purpose of receiving a corresponding elastically deformable annular protrusion on the outer surface of a polyethylene bearing insert (not shown). Annular groove 34 in cooperation with the annular protrusion of the polyethylene bearing insert serves to axially retain the bearing insert within acetabular shell 10.

A plurality of notches 36 are circumferentially spaced about rim 26 to receive a radial protrusion (not shown) of the polyethylene bearing insert to retain the bearing insert against rotation.

An annular groove 38 having a flat end wall 40 and an annular side wall 42 is located in inner surface 14. Annular groove 38 serves to receive a positioning tool for holding and positioning acetabular shell 10 within the acetabulum during implantation.

A dome hole 44 is centered at the apex 24 in coaxial alignment with axis 16. Dome hole 44 is bounded by a substantially cylindrical side wall 46, a frusto-conical portion 47 extending from said side wall 46 to the inner hemispherical surface 14, and a flat end wall 48. Dome hole 44 does not extend through shell wall 18 to the exterior of acetabular shell 10, but rather is closed at one end by end wall 48 and is open at the other end toward the interior of acetabular shell 10. Dome hole 44 receives a corresponding cylindrical protrusion on a polyethylene bearing insert to stabilize the insert against displacement relative to acetabular shell 10. Dome hole 44 can also be employed to receive a positioning tool.

Referring in particular to FIGS. 1 and 3, screw hole 50 is described. In the embodiment illustrated herein, there are preferably two screw holes 50 provided in addition to dome hole 44, as is evident in FIG. 2. It should nevertheless be understood that the present invention is also useful in connection with any number of screw holes that may be distributed across acetabular shell 10 to provide the implanting physician with a selection of sites for inserting a bone screw, with the understanding that not all of the screw holes so provided would necessarily be used during any particular implantation. Screw hole 50 is bounded by a substantially cylindrical side wall 52, a spherical surface 54 extending from said side wall 52 to the inner hemispherical surface 14, and a flat end wall 56. Surface 54 is concave toward the interior of acetabular shell 10 and has its center 58 located radially inwardly of inner surface 14. Surface 54 as shown is spherically configured for the purpose of engaging a spherical undersurface of the head of a bone screw. Alternatively, surface 54 could be conically configured for engaging a screw having a head with a conical undersurface. Screw hole 50 does not extend through shell wall 18 to the exterior of acetabular shell 10, but rather is closed at one end by end wall 56 and is open at the other end toward the interior of acetabular shell 10.

As best shown in FIG. 3, cylindrical side wall 52 and flat end wall 56 intersect to define a flat bottomed blind hole that extends almost, but not quite, to a circular intersection with outer hemispherical surface 12. The result is that the portion of shell wall 18 that is partially penetrated by screw hole 50 is quite thin, leaving a thin integral seal 62 that completely occludes screw hole 50 and absolutely prevents passage of wear debris from the interior of acetabular shell 10 through screw hole 50, so long as seal 62 remains intact and integral with shell wall 18. It should be noted that screw hole 50 is conveniently formed by machining a flat bottomed cylindrical hole in shell wall 18 from the interior of acetabular shell 10, with the cylindrical hole having a diameter corresponding to cylindrical side wall 52, followed by machining a spherical countersink corresponding to spherical surface 54. The flat bottom of the cylindrical hole is machined to within about 0,25 mm (0.010 inch) of outer hemispherical surface 12 at the periphery 64 of the flat end wall 56. Because outer hemispherical surface 12 remains spherical where it overlies screw hole 50, seal 62 is thicker at its center than at its edges. Thus, there is formed a screw hole seal 62 that is integral with shell wall 18, but is thinnest at its peripheral circular edge 64 located at the cylindrical side wall 52 of screw hole 50. The thickness of seal 62 at its circular edge is selected, taking into consideration the characteristics of the material of which shell wall 18 is made, such that seal 62 can be broken out of shell wall 18 by application of appropriate force, such as with a punch, to render blind screw hole 50 into a through screw hole for receipt of a bone screw. With titanium as the material of which shell wall 18 is made, it is desirable that the thickness at the circular edge of seal 62 be less than about 0,64 mm (0.025 inches). Preferably, the thickness of seal 62 at the circular edge should be about 0,25 mm (0.010 inches). If the physician chooses not to select a particular screw hole 50 for receipt of a bone screw, seal 62 is simply left undisturbed to completely occlude that screw hole and eliminate it as a possible path for migration of debris out of acetabular shell 10. Because seal 62 is thicker at its center, it tends to break out cleanly from shell wall 18 at its peripheral circular edge 64 and leave a circular opening aligned with cylindrical side wall 52. Seal 62 is most conveniently made thinner at its edge by machining the bottom of the screw hole flat while leaving the outer surface of the acetabular shell spherical, but other configurations of the screw hole and outer surface of the shell that provide the same result of the seal preferentially breaking out at its edge will be apparent to those of ordinary skill in the art.

It should also be understood that the foregoing description of the break out seals 62 associated with screw holes 50 applies as well to a break out seal associated with dome hole 44. In addition, the break out seal arrangement as described herein can be used in connection with any hole in an acetabular shell where it is desired to provide the choice of occluding or opening the hole, regardless of the intended purpose of the hole.

While the present invention has been illustrated and described with particularity in terms of a preferred embodiment, it should be understood that no limitation of the scope of the invention is intended thereby. The scope of the invention is defined only by the claims appended hereto. It should also be understood that variations of the particular embodiment described herein incorporating the principles of the present invention will occur to those of ordinary skill in the art and yet be within the scope of the appended claims.

## Claims

1. An acetabular shell (10) that can be used with or without bone screws, comprising:
a generally hemispherical cup-shaped shell having an inner surface (14) and an outer surface (12) defining a shell wall therebetween, said shell wall (18) having at least one blind hole (50) in the inner surface thereof, said blind hole (50) terminating sufficiently close to the outer surface of said shell to leave a thin seal (62) integral with said shell wall and occluding and separating said blind hole from the exterior of said shell, the seal having at least a portion thereof that is sufficiently thin that the seal can be broken out of the shell wall (18) to render said blind hole (50) as a through hole communicating from the interior to the exterior of said shell.

2. The acetabular shell of claim 1, wherein said seal (62) is thinnest at its peripheral edge such that said seal tends to break out cleanly and whole at its peripheral edge.

3. The acetabular shell of claim 2, wherein said blind hole (50) has a flat end wall.

4. The acetabular shell of claim 3, wherein said outer surface overlying said blind hole (50) is spherical.

5. The acetabular shell of claim 1, wherein said blind hole (50) is configured to receive a bone screw therethrough after said seal (62) is broken out.

6. The acetabular shell of claim 1, wherein said thin portion of said seal (62) is less than about 0,64 mm (.025 inches) in thickness.

7. The acetabular shell of claim 6, wherein said thin portion of said seal (62) is about 0,25 mm (0.010 inches) in thickness.

8. The acetabular shell of claim 2, wherein said peripheral edge of said seal (62) is less than about 0.64 mm (.025 inches) in thickness.

9. The acetabular shell of claim 8, wherein said peripheral edge of said seal (62) is about 0,25 mm (0.010 inches) in thickness.

10. The acetabular shell of claim 1, wherein said blind hole (50) is a machined hole in the shell wall (18).

## Patentansprüche

1. Hüftgelenkpfanne (10), die mit oder ohne Knochenschrauben eingesetzt werden kann und die umfaßt:
eine im allgemeinen halbkugelartige schalenförmige Pfanne mit einer Innenfläche (14) und einer Außenfläche (12), die eine Pfannenwand dazwischen bilden, wobei die Pfannenwand (18) wenigstens ein Blindloch (50) in der Innenfläche aufweist, wobei das Blindloch (50) nahe genug an der Außenfläche der Pfanne endet, um einen dünnen Verschluß (62) zu belassen, der eine Einheit mit der Pfannenwand bildet und das Blindloch abschließt sowie von der Außenseite der Pfanne trennt, wobei der Verschluß wenigstens einen Abschnitt aufweist, der so dünn ist, daß der Verschluß aus der Pfannenwand (18) herausgebrochen werden kann, um aus dem Blindloch (50) ein Durchgangsloch zu machen, das Verbindung von der Innenseite der Pfanne nach außen herstellt.

2. Hüftgelenkpfanne nach Anspruch 1, wobei der Verschluß (62) an seinem Umfangsrand am dünnsten ist, so daß der Verschluß sauber und als Einheit an seinem Umfangsrand bricht.

3. Hüftgelenkpfanne nach Anspruch 2, wobei das Blindloch (50) eine flache Abschlußwand aufweist.

4. Hüftgelenkpfanne nach Anspruch 3, wobei die Außenfläche über dem Blindloch (50) kugelförmig ist.

5. Hüftgelenkpfanne nach Anspruch 1, wobei das Blindloch (50) so aufgebaut ist, daß es eine Knochenschraube aufnimmt, wenn der Verschluß (62) herausgebrochen wurde.

6. Hüftgelenkpfanne nach Anspruch 1, wobei der dünne Abschnitt des Verschlusses (62) weniger als 0,64 mm (0,025 Inch) dick ist.

7. Hüftgelenkpfanne nach Anspruch 6, wobei der dünne Abschnitt des Verschlusses (62) ungefähr 0,25 mm (0,010 Inch) dick ist.

8. Hüftgelenkpfanne nach Anspruch 2, wobei der Umfangsrand des Verschlusses (62) weniger als 0,64 mm (0,025 Inch) dick ist.

9. Hüftgelenkpfanne nach Anspruch 8, wobei der Umfangsrand des Verschlusses (62) ungefähr 0,25 mm (0,010 Inch) dick ist.

10. Hüftgelenkpfanne nach Anspruch 1, wobei das Blindloch (50) ein maschinell in der Pfannenwand (18) hergestelltes Loch ist.

## Revendications

1. Coque (10) acétabulaire qui peut être utilisée avec ou sans vis à os, comprenant :
une coque en forme de cuvette de forme générale hémisphérique ayant une surface intérieure (14) et une surface extérieure (12) qui définissent entre elles une paroi de coque, ladite paroi de coque (18) ayant au moins un trou borgne (50) dans sa surface intérieure, ledit trou borgne (50) se terminant suffisamment prés de la surface extérieure de ladite coque pour laisser un mince opercule (62) d'une seule pièce avec ladite paroi de la coque et qui obture ledit trou borgne et le sépare de l'extérieur de ladite coque, l'opercule possédant au moins une portion qui est suffisamment mince pour que cet opercule puisse être arraché de la paroi (18) de la coque pour transformer ledit trou borgne (50) en un trou traversant qui établit la communication entre l'intérieur et l'extérieur de ladite coque.

2. Coque acétabulaire selon la revendication 1, dans laquelle ledit opercule (62) est le plus mince au droit de son bord périphérique, de sorte que ledit opercule tend à s'arracher proprement et entièrement le long de son bord périphérique.

3. Coque acétabulaire selon la revendication 2, dans laquelle ledit trou borgne (50) possède une paroi terminale plate.

4. Coque acétabulaire selon la revendication 3, dans laquelle ladite surface extérieure qui recouvre ledit trou borgne (50) est sphérique.

5. Coque acétabulaire selon la revendication 1, dans laquelle ledit trou borgne (50) est configuré pour recevoir une vis à os qui la traverse après que ledit opercule (62) a été arraché.

6. Coque acétabulaire selon la revendication 1, dans laquelle ladite portion mince dudit opercule (62) est d'une épaisseur de moins d'environ 0,64 mm (0,025 pouce).

7. Coque acétabulaire selon la revendication 6, dans laquelle ladite portion mince dudit opercule (62) est d'une épaisseur d'environ 0,25 mm (0,010 pouce).

8. Coque acétabulaire selon la revendication 2, dans laquelle ledit bord périphérique dudit opercule (62) est d'une épaisseur de moins d'environ 0,64 mm (0,025 pouce).

9. Coque acétabulaire selon la revendication 8, dans laquelle ledit bord périphérique dudit opercule (62) est d'une épaisseur d'environ 0,25 mm (0,010 pouce).

10. Coque acétabulaire selon la revendication 1, dans laquelle ledit trou borgne (50) est un trou usiné dans la paroi (18) de la coque.
